# EUROPEAN PATENT SPECIFICATION

(11) **EP 2 739 270 B1**
(45) Date of publication and mention of the grant of the patent: **01.01.2020**
(21) Application number: 12819632.6
(22) Date of filing: 01.08.2012
(51) Int. Cl.: A61K 9/48, A61K 31/721, A61K 31/738

(54) **NON-DIGESTIBLE CAPSULES FOR THE DELIVERY OF FLUID ABSORBING MATERIALS**
UNVERDAULICHE KAPSELN ZUR VERABREICHUNG FLUIDABSORBIERENDER MATERIALIEN
CAPSULES NON DIGESTIBLES UTILISÉES POUR L'ADMINISTRATION DE MATÉRIAUX ABSORBANT LES FLUIDES

(30) Priority: 01.08.2011 US 201161513923 P; 15.08.2011 US 201161523787 P
(43) Date of publication of application: 11.06.2014
(73) Proprietor: HB Biotechnologies Corporation, New York, NY 10014 (US)
(72) Inventor: BERGER, Danielle, New York New York 10014 (US)
(74) Representative: Patentanwälte Bressel und Partner mbB
(86) International application number: PCT/US2012/049209
(87) International publication number: WO 2013/019891

(56) References cited:
- WO-A1-90/09168
- WO-A2-2007/017842
- WO-A2-2009/049105
- US-A- 4 462 982
- US-A- 4 470 975
- US-A- 4 663 148
- US-A- 5 169 638
- US-A- 5 264 223
- US-A- 5 770 224
- US-A1- 2004 105 895
- US-A1- 2010 215 732
- US-A1- 2011 171 298
- US-B2- 6 908 609

## Description

### BACKGROUND OF THE INVENTION

In certain diseases, particularly in kidney diseases, fluid retention within an animal's body presents serious difficulties. With total failure of the renal system, fluid build-up in the body, called edema, can lead to an accumulation in the blood of constituents or serum toxins normally eliminated in the urine, producing a severe toxic condition. This toxic condition can lead to death. The conventional treatment for diseases of this nature is periodic hemodialysis, where artificial kidney machines eliminate water and toxins from the body.

The cost of dialysis is exceedingly high and the availability of dialysis machines is not nearly as great as is convenient for both the practitioner and the patient involved. Additionally, the patient undergoing dialysis may suffer from significant physiological and mental discomfort. For these reasons, it is highly desirable to limit the frequency of dialysis to the minimum number of treatments necessary to preserve health.

Dialysis accomplishes two major objectives, viz. it removes both water and toxins from the body. The toxins are, primarily, substances resulting from protein metabolism. By proper control of the diet of the patient, particularly with regard to the amount of protein in the patient's diet, the necessary frequency of dialysis for removal of these toxins can be considerably reduced as compared to the frequency required with an unrestricted diet. However, unless the patient's consumption of water is severely limited, frequent hemodialysis is still necessary for the removal of water.

Restricting the patient's intake of water is generally very difficult, since patterns of water consumption are often deeply ingrained and changing these patterns may result in severe physical and mental discomfort to the patient. Many patients are not able to restrict their water intake to the minimum necessary for substantial reduction in the frequency of required dialysis. Accordingly, if a product and/or method were provided for removal of water from the body, then frequency of dialysis could be substantially reduced. Dialysis would still be required for the periodic removal of protein derived toxins, but the frequency of dialysis for this purpose would be far less than the frequency normally required for the removal of both water and protein derived toxins.

US 6,908,609 B2 discloses a method and material for removing fluid from the intestinal tract of a host by ingesting an enterically coated non-systemic, non-toxic, non-digestible, water absorbing polymer which absorbs fluid while passing through the intestinal tract.

### SUMMARY OF THE INVENTION

The present invention provides an orally consumable medical product according to claim 1, which is a product, system, or means for delivering a fluid-absorbing material to a subject suffering from excess fluid retention, whereby the product, system, or means reduces the retention of fluid in said subject.

It is an object of this invention that the fluid-absorbing material is ingested and delivered to the digestive tract as a product in capsule or capsular-like form.

It is a further object of this invention that the capsule or capsular-like form is constructed of substantially nitrogen-free material, comprising less than 4% nitrogen, which is preferably a non-digestible or non-metabolizable material.

It is yet another object of this invention that when the capsule or capsular-like form, which is made of non-digestible or non-metabolizable material or materials, is ingested, is dispersed, and the material or materials contributes little to no protein to the bloodstream.

It is an object of this invention that the fluid-absorbing material is a cross-linked polymer material in the form of a fiber, particle, granule, powder, or a combination thereof.

It is a further object of this invention that fluid-absorbing material is released from the capsule in a location, such as the stomach or intestine, that will allow for the absorption of fluid, thereby reducing the accumulation of excess fluid.

It is yet another object of this invention that the fluid-absorbing material, itself, is not digested or metabolized. In other words, the fluid-absorbing material does not undergo chemical or physical breakdown into its constituent parts and is excreted or expelled from the body with little to no degradation. The fluid absorbing material is insoluble.

It is still another object of this invention that the fluid-absorbing material is an insoluble hydrophilic, cross-linked polymer having high or super absorbent properties.

It is another object of this invention that the fluid-absorbing material absorbs all molecules by means of 3D entrapment, or any means other means known for absorbing and retaining fluids.

It is another object of this invention that the fluid absorbing material absorbs or adheres molecules that are smaller than the largest molecule of the absorbing materials or molecules. These might include for example toxins, bacteria, viruses, ions, solutes, or any substances dissolved or admixed with the fluid being absorbed or retained.

The present invention also provides a method of treating the build up of fluids or edema and thereby reducing the patient's need for fluid relieving treatments or dialysis.

It is an object of this invention to administer to a subject in need thereof a capsule or capsular-like form that, when ingested, breaks, splits, dissolves, disintegrates, or ruptures in a part of the gastrointestinal tract, thereby releasing its fluid-absorbing material contents. The fluid-absorbing material contents are capable of absorbing excess fluids in the subject.

It is further an object of this invention to treat edema with reduced reliance on hemodialysis due to the decreasing rate of renal elimination of water.

It is a another object of this invention to treat edema with reduced reliance on hemodialysis by increasing intestinal water elimination.

It is still another object of this invention to provide an orally consumable medical product of the invention for a new treatment for diseases characterized by an abnormal excess accumulation of fluid within the body, such as, congestive heart failure, cirrhosis of the liver, nephrosis, and other renal diseases associated with fluid retention.

It is also another object of this invention to treat, with an orally consumable medical product of the invention, diseases associated with kidney or liver failure by assisting in the removal of toxins that accumulate in the body. The treatment may include the removal of excess ions or serum toxins, such as but not limited to potassium or ammonium.

It is yet another object of this invention to treat diseases such as hyperkalemia, uremia, or hyperammonemia comprising the administration of a capsule of the instant invention.

By this invention fluid may be removed from the gastrointestinal tract, gastrointestinal transit time reduced, and caloric intake decreased.

Weight control, weight loss, or appetite suppression can be reached by administering to a subject a capsule of the instant invention.

Another object of this invention is to provide a fluid-absorbing material with some fibrous characteristics to act as a bulk forming agent.

The delivery of certain insoluble hydrophilic, cross-linked polysaccharides in an encapsulated and ingestible form are useful pharmaceutical agents in diverting the route of water elimination from the renal route to the gastrointestinal route, thereby removing excess water from the body by the gastrointestinal route. These properties are of specific therapeutic value in the treatment of water intoxication in chronic renal failure, in reducing the frequency of hemodialysis, and in the treatment of other forms of fluid retention such as congestive heart failure, cirrhosis of the liver, and other disorders associated with refractory swelling. These pharmacological properties also provide a means of reducing caloric intake, and hence useful in the treatment of conditions such as obesity.

### BRIEF DESCRIPTION OF THE DRAWINGS

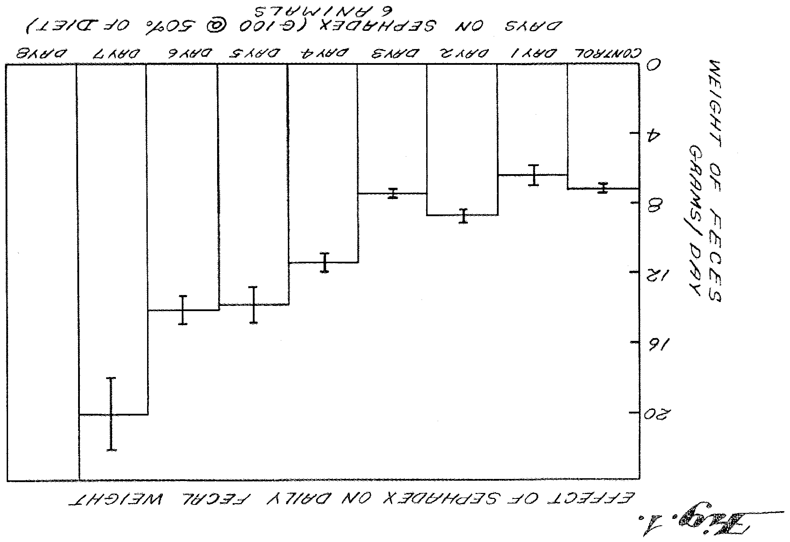

Figure 1 illustrates the effect of treatment with insoluble, hydrophilic, cross-linked dextrans according to the invention on the weight of feces eliminated by treated rats. It will be noted that the weight of feces of rats treated according to the present invention increases significantly when compared to rats which did not receive treatment.

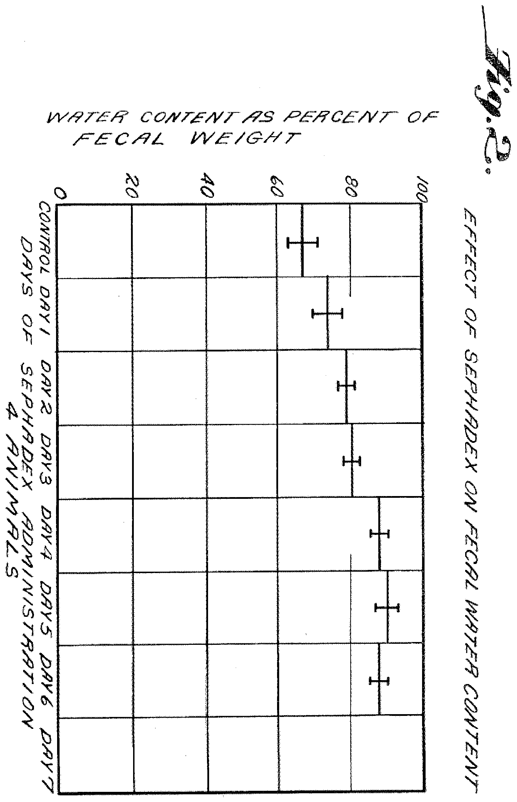

Figure 2 shows the effect of treatment with insoluble, hydrophilic, cross-linked dextrans according to the invention on water content as a percentage of the weight of feces of rats treated according to the present invention. It will be noted that the water content of feces of rats treated according to the present invention is significantly higher than that of untreated rats.

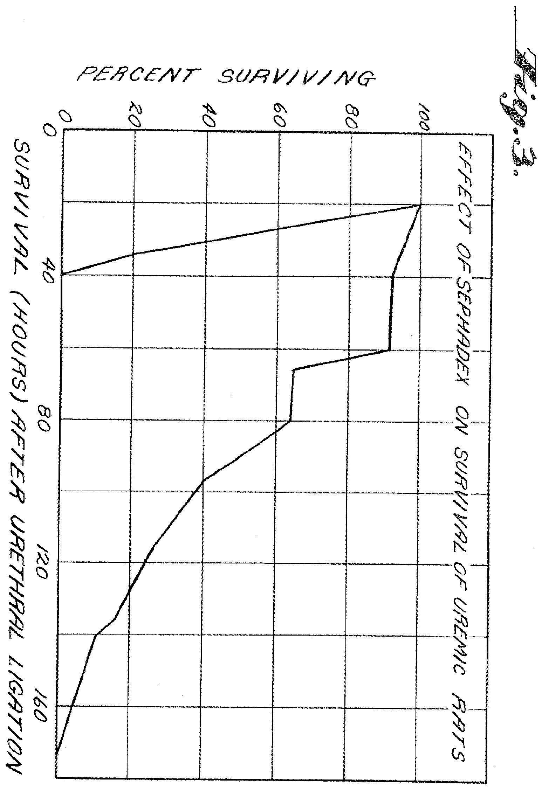

Figure 3 shows the effect of the use of insoluble, hydrophilic, cross-linked dextrans according to the present invention on the survival of rats with urethral ligation. It will be noted that rats treated with insoluble, hydrophilic, cross-linked dextran according to the present invention survived significantly longer than rats which received no treatment.

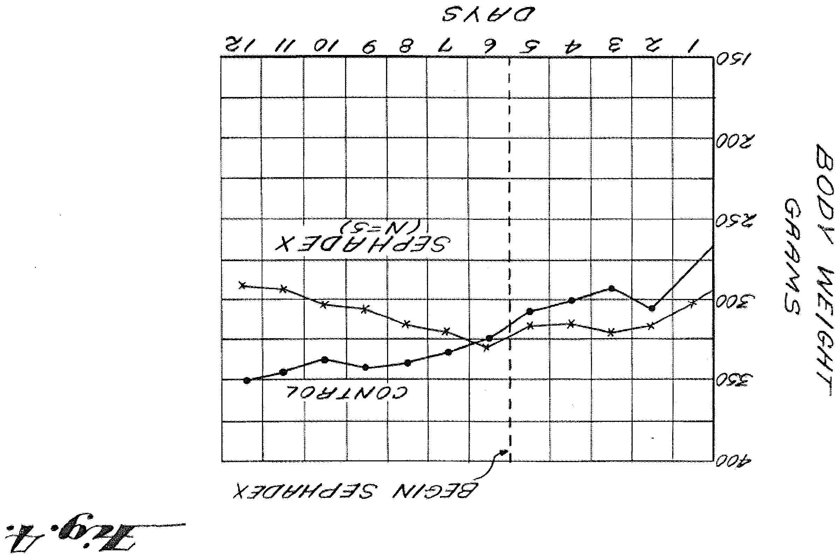

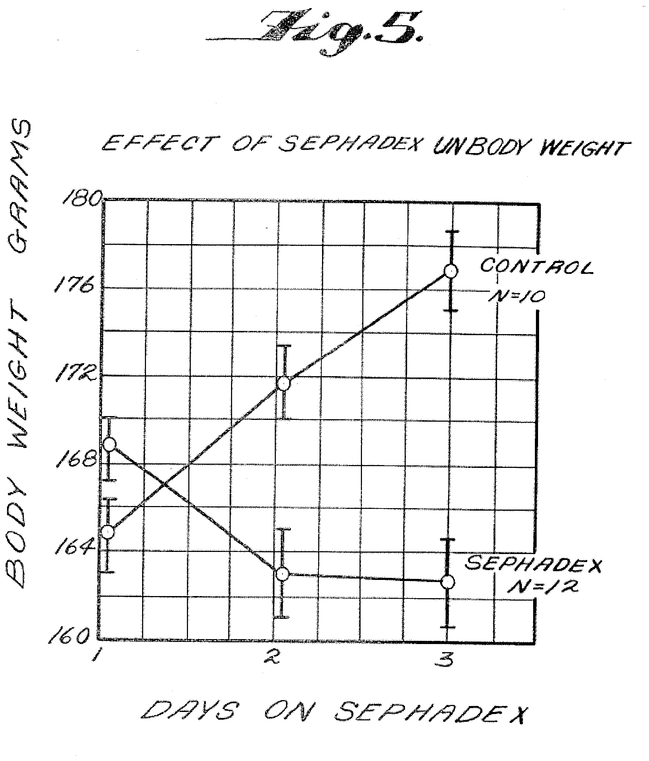

Figures 4 and 5 illustrate the effect of administering insoluble, hydrophilic, cross-linked dextran according to the invention on the body weight of rats. It will be noted that shortly after the beginning of treatment the body weight of treated rats began to drop, becoming significantly lower than the body weight of untreated rats.

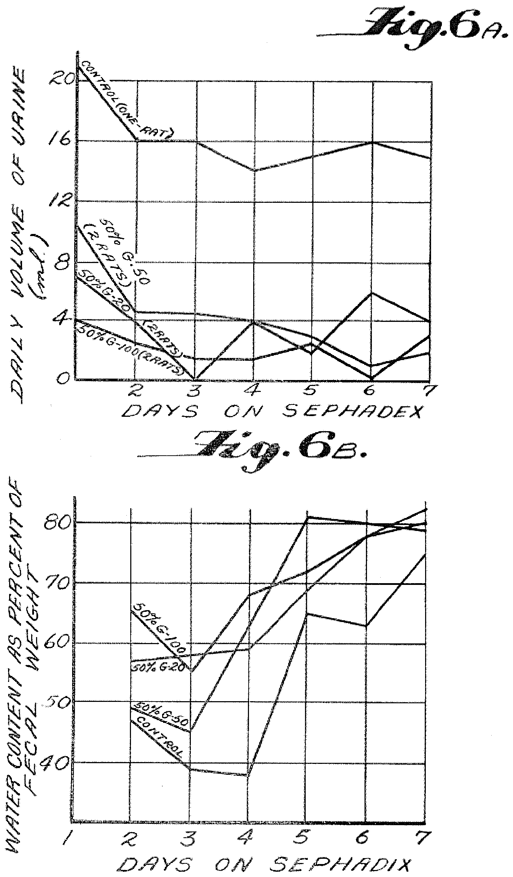

Figures 6A and 6B illustrate the effect of treatment on the volume of urine and water content of the feces of rats treated according to the present invention. It will be noted that the volume of urine of rats treated according to the present invention was substantially lower than the volume of untreated rats, and that at the same time, the water content of the feces of treated rats increased compared to the water content of the feces of untreated rats.

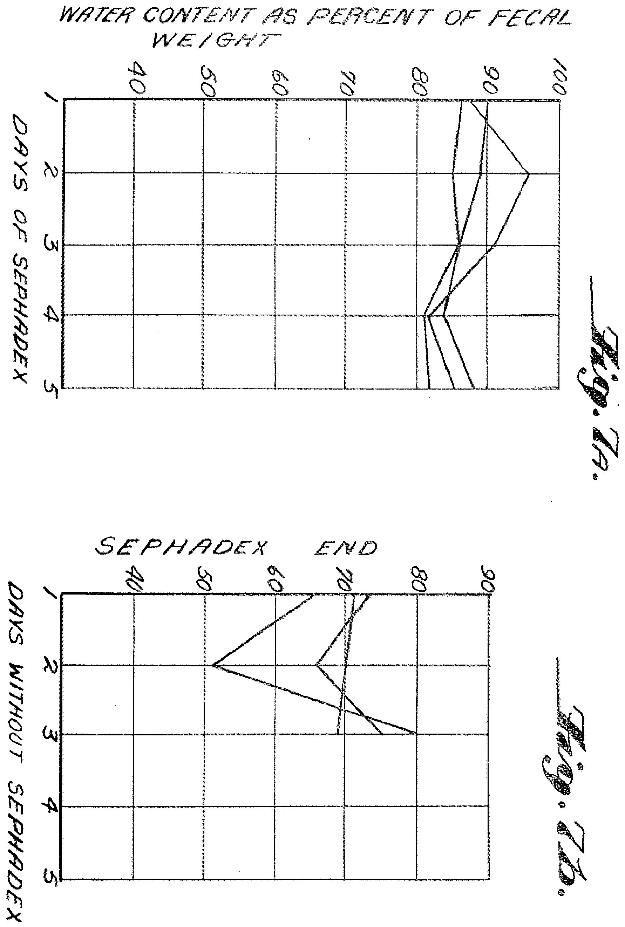

Figures 7A and 7B illustrate the effect of treatment of various concentrations of the preferred insoluble, hydrophilic, cross-linked dextrans of the present invention on the volume of urine of treated rats and the water content of the feces of treated rats. After treatment was ended, the volume of urine and water content of the feces of the rats were again measured and are also illustrated.

### DETAILED DESCRIPTION OF THE INVENTION

It has been found in accordance with the present invention that the ingestion of a capsule or capsular like form (herein "capsules") containing large quantities of an insoluble hydrophilic fluid-absorbing material has the ability to bind, entrap, and/or absorb large quantities of fluid, such as water, in the gastrointestinal tract. The capsules may be ingested as an intact unit that releases, ruptures, breaks, disintegrates, or splits open upon reaching a desired location within the gastrointestinal tract. The capsules of the present invention, in one embodiment, are capable of diverting the mode of fluid and waste elimination from the renal route to the gastrointestinal route.

The contents of the capsules, which comprise insoluble fluid-absorbing materials or hydrophilic materials, are released and begin absorbing, binding or entrapping excess fluid as it transverses the gastrointestinal tract. The capsule contents are designed to bind and/or absorb excess fluid in the subject, followed by elimination or by passage from the alimentary canal as solid waste. The capsule is therefore useful in the treatment of a variety of diseases in which abnormal or excess fluid retention may cause medical problems, such as, but not limited to edema. Further, the capsule of the instant invention may also be useful in the elimination of excess serum toxins that accumulate in the body due to liver or kidney failure.

Without being bound to any specific mechanistic theory, the capsules are designed to remove excess fluid by releasing large quantities of an insoluble, hydrophilic material in a subject, whereby the released material binds, entraps, and/or absorbs excess fluid and the fluid is excreted as a solid, without renal involvement. The fluid absorbing material may bind, entrap, or absorb the excess fluid through a variety of mechanisms, such as but not limited to 3-D entrapment within the physical spaces of the hydrophilic material. The capsules are orally delivered to, administered to, or ingested by a patient suffering from diseases associated with the abnormal accumulation or retention of fluid or water in the body. Alternatively, the capsule may be orally delivered to, administered to, or ingested by a patient suffering from insufficient, abnormal, or malfunctioning kidney or liver function.

The capsule of the present invention is also useful in the delivery of the fluid absorbing material that acts as a bulking agent. Accordingly, the fluid absorbing material that is delivered by the capsule may be useful in the treatment of irritable bowel disease, non-insulin dependent diabetes, high cholesterol, lowering triglycerides, or any other issue where fiber is known to be beneficial. Other useful treatments with the capsule of the present invention may include weight control, weight loss, or appetite suppression.

The capsule is designed to release its fluid-absorbing contents as it passes through the digestive system. Water passes from the body into the lumen of the gastrointestinal system where the fluid-absorbing material entraps the fluid and removes its from the body. As is known, water and urea readily penetrate the lining of the lumen, and by using the product and/ or the method of the present invention, water and urea are continuously held within the lumen by the released insoluble, hydrophilic, materials of the present invention. By entrapping water and urea in the lumen of the gastrointestinal system with the insoluble, hydrophilic materials released from the capsules of the present invention, water passing from the body into the gastrointestinal system cannot be taken back into the body, which results in the net decrease of water content from the body.

The term "product", "product" or some grammatical derivative thereof, as used herein, refers to a device, drug, non-ionic substance, or variations thereof, useful for the delivery of a fluid absorbing material to a subject. In an alternative embodiment, the "product" may be an ionic substance or may additionally comprise an ionic substance in addition to the non-ionic substance.

### The capsules

Conventional capsules are metabolized or digested in the body. These capsules tend to contain or provide an additional source of protein and/or metabolic waste to the body. Patients suffering from diseases characterized by the excess build up of fluids typically require procedures, such as but not limited to dialysis, to aid in the removal of water, toxins, protein build up, metabolic waste, and/or blood borne waste from the body. Accordingly, a capsule constructed of a proteinacious component would further compound the symptoms in these patients. For example, a protein based capsule may be digested in the stomach and result in the release of proteins, amino acids, and other constituent parts that contribute to the nitrogen waste level, which may be toxic to the body upon accumulation. Accordingly, the ingestion of such a capsule may contribute and/or exacerbate the underlying condition suffered by the patient, especially if numerous capsules are ingested on a daily basis. Consequently, a conventional capsule which may comprise fluid-absorbing materials would be counterproductive in these types of patients, because the remedy that is intended to alleviate the problem would simultaneously contribute to the problem. Most fluid-absorbing materials are designed with the specific purpose of binding to fluid while not having the capacity to absorb or bind to proteins. Accordingly, the fluid-absorbing materials do not counteract the contribution of additional proteins and/or metabolic waste provided by the digested capsule. Accordingly, this invention uses a product comprising a capsule for providing fluid-absorbing materials in which capsules are prepared from nitrogen-free components and/or components that are not easily digested and/or metabolized.

As can be appreciated from the foregoing, the capsules according to the invention must be capable of releasing its contents within any locus of the digestive system. The digestive system is commonly understood to begin at the mouth and end at the anus, wherein the actual digestive process begins in the mouth but is completed in the small intestine. Accordingly, the capsules are preferably designed to release their contents in the stomach and/or small intestine. Further, the capsules, while capable of releasing its contents in the digestive system, are preferably not themselves capable of being digested and/or metabolized. Thus, the preferred capsule must be prepared from a material and in a manner which allows the release of its contents while contributing little to no protein content and/or additional metabolic waste to the subject. Effectively, the capsules may be degraded but not absorbed in the body.

The capsules used in the present invention are designed to release its contents through various mechanisms commonly understood to those of skill in the art. These mechanisms may include, but are not limited to, rupturing, breaking, bursting, splitting open, disintegrating, and/or dissolving without contributing any additional serum toxins or proteins to the body. A typical mechanism for the release of the contents may, for example, be described as a disintegration of the capsule upon contact with water and/or acid.

An alternative mechanism for content release may include release by swelling. For example, the capsule may be constructed of a semi-permeable coating that allows for the influx of water and/or acid into the capsule. The permeability of the outer layer causes the inner contents to absorb water and/or acid resulting in the swelling of the outer layer, ultimately resulting in the release of the internal contents by breaking the outer coating.

Given the variation in the pH ranges along the digestive tract, the capsule may also be designed to breakup, disintegrate, or become permeable in a particular pH range. This effectively controls the location of content release along the digestive tract because of the variation in pH along its length. For example, if a release of the inner contents is desired in the stomach, a capsule may be constructed of materials capable of disintegrating in a pH range of about 1-3. However, if the contents of the capsules are desired to be released in the small intestine, the capsule may be constructed of materials capable of disintegrating and/or permeable to fluids in the pH range of about 5-8. Accordingly, the capsule may be designed to release its contents in any pH range of about 1-14. Additionally, a capsule that is designed to be sensitive to pH may be prepared from a one piece capsule that dissolves or as a two piece capsule that splits or breaks open.

The capsules of the present invention may also be administered in a variety of therapeutic dosages suitable for the removal of excess fluid in a subject. Depending on the applicable disease state treated, the capsule may comprise an unlimited range of fluid absorbing material. The limit on the amount of fluid absorbing material contained within the capsule will be dictated by toxicological, environmental, and physical factors associated with the ingestion and manufacture of an orally consumable capsule. Those of skill in the art will recognize these limitations and will be capable of adjusting the dosages required for the treatment or manufacture of a particular disease state. In any event, the capsule may contain, for example, in the range of approximately 10 milligrams to approximately 20,000 milligrams or 20 grams of the fluid absorbing material. A preferred amount of fluid absorbing material contained in each capsule is about 1, 2, 3, 4, 5, 6, 7, 8, 9, 10, 11, 12, 13, 14, 15, 16, 17, 18, 19, or 20 grams. Those of skill in the art will also understand that the number of capsules administered to the patient may be dictated by the severity of the disease as well as by the concentration of fluid absorbing material encapsulated within the capsule or capsular product. As an example, those of skill in the art may administer to a subject suffering from a disease of excess fluid retention an amount of a capsule comprising a fluid absorbing material in the range of 10 milligrams to about 20,000 milligrams. Accordingly, those of skill in the art will understand that the amount of fluid absorbing material administered to the patient is determined by the patient's amount of fluid intake.

The material composition of the capsules used in the present invention is substantially nitrogen-free and preferably non-digestible and/or non-metabolizible. Accordingly, the capsules will release their contents, but will not break-down into their chemical constituent parts. As understood by those of skill in the art, the term "digest," "digestion," or grammatical variants thereof, means the mechanical or chemical break down of a substance into smaller parts which can be absorbed into the body. Accordingly, the capsules of this invention should preferably be prepared from inert materials. These inert materials may include, for example, weakened plastics, rubbers, silicone, digestion-resistant starches and fibers, and/or gelatinous materials all of which contain little to no protein or other nitrogen-containing material. As understood by those of skill in the art, the term "substantially nitrogen-free" means a substance or material that comprises less than 4% nitrogen, preferably the substance or material that comprise less than 1% nitrogen. While not being bound to any particular theory, inert materials may allow the influx of acids and/or water into the capsules causing the materials to loosen and/or soften, thereby allowing the capsule to release its contents. The remaining capsule and its parts remain intact or degrade into materials that are not preferably absorbed into the body and/or disseminated into the blood stream, but are excreted as part of the solid waste. Alternatively, capsules made of inert materials may release its contents by allowing the influx of water and/or acid thereby causing the capsule to release its contents upon breaking open caused by swelling. Again, the remaining capsule parts are either intact or breakup into materials that are indigestible or non metabolizible and which are excreted as part of the solid waste.

Other possible types of capsules include but are not limited to capsule products prepared from a silicone-rubber casing; pulsincap or time release capsules; pullulan or all natural capsules; hydromellose capsules; cellulose based capsules; alginate-based; and carbohydrate-based capsules.

Silicone-rubber based capsule casing are fully described in, for example, FR 2007452. These capsules may be prepared from two pieces or as single piece capsules. If the capsules are two pieces or two halves, the capsule may be designed to release its contents by disintegrating or unbinding of the two halves to release its contents.

Pulsincap or time release capsules are designed to house the insoluble fluid-absorbing material as a drug reservoir. The capsule comprises, for example, a single opening sealed with an insoluble plug which may be released, disintegrated, erodible, congealable, or enzymatically activate upon entry into a particular environment, such as pH. The insoluble plug may be made of a swellable hydrogel or any other material which allows the for the permeation, release, or spillage of content from the capsule. The length, position of insertion, and type of material used to prepare the plug dictates the release time of the its contents. Accordingly, the contents of the capsule may be designed such that the plug is inserted at a particular depth, position, and made of a type of material which allows for the release of the fluid-absorbing material in the stomach or in the small intestine.

The term "hydrogel" is understood by those of skill in the art. It is conventionally understood to encompass a network of hydrophilic polymer chains, natural or synthetic polymer chains. Those of skill in the art will also appreciate that a hydrogel is a form of super absorbent polymer. Accordingly, the hydrogel plug envisioned in this invention may be prepared from, for example, polymethacrylates, hydropropylmethyl cellulose, polyvinyl alcohol, polyvinyl acetate, polyethylene oxide, saturated polyglycolated glycerides, glyceryl monooleates, pectin, sodium polyacrylate, acrylate polymers, copolymers with an abundance of hydrophilic groups, agarose, methylcellulose, hyaluronan, and other naturally derived polymers.

Pullulan capsules are water soluble and completely biodegradable two-piece capsules made from vegetable derived polysaccharides. The polysaccharides are produced through a fermentation process that results in a capsule which is impermeable to oxygen transmission. These types of capsules are recommended for encapsulating oxidation sensitive materials or contents. These all-natural, non-animal capsules are suitable for those who have cultural or dietary requirements based on the non-consumption of animal parts. Accordingly, these types of capsules would be suitable for patients who are vegetarians, diabetics, vegans, and those with restricted diets. US Patent Application Publication US20070141137 provides a description of these types of capsules. A preferred pullulan capsule used in the present invention is Capsugel's® NPcaps.

Hydromellose capsules are also non-animal based capsules that are suitable for those with cultural and/or dietary restrictions. These capsules are suitable for moisture-sensitive contents and are resistant to cross-linking. A preferred hydromellose capsule used in the present invention is Capsugel's Vcap and/or Vcap plus.

Cellulose-based capsules are prepared from a cellulose or cellulose like material and do not contain proteins. As a results of the construction material, these capsules are also indigestible, resulting in little or no bioavailability/contribution to a patient.

Alginate-based capsules are non-animal, non-protein based, indigestible capsules made from natural polymers extracted from seaweed. Because of the encapsulation process, capsules made from this material are thinner and are capable of containing larger quantities of fluid absorbing materials. Consequently, capsules of this type when utilized in the present invention will allow the subject to reduce the number of doses taken per day. Another advantage of this material is that the capsule is inherently enteric, thereby allowing the release of the fluid absorbing material in a more alkaline environment, such as the small intestine. Various processes and methodologies for encapsulating the fluid absorbing material within an alginate capsule are well known, see for example U.S. Patent 5,942,266, U.S. Patent 7,766,637, U.S. Patent 7,972,620, and U.S. Patent Application Publication 2010/0266848.

Carbohydrate-based capsules also provide an alternative to animal-based capsular materials. These are typically made of biodegradable carbohydrates derived from plants. These carbohydrates may include for example starches that are indigestible in humans or resistant starches that are not absorbed in the small intestine.

### The fluid-absorbing material

The fluid-absorbing materials of this invention may be in fibrous, particulate, powder, crystalline, gel, or granular forms. The materials used to prepare the fluid-absorbing materials of this invention may be derived from natural or synthetic polymers. The materials may preferably be polyelectrolyte and non-polyelectrolyte polymers. These include, *inter alia,* amine containing, carboxylate-containing, sulfonate-containing, polyacrylic, polyvinyl, and/or polysacchrides polymers such as dextrans, Sephadex, Sephacryl, Superdex, Superose, Sephacel, Sepharose, or any derivative thereof. The fluid absorbing material of the present invention will be capable of absorbing large quantities of fluid relative to its own mass. Accordingly, the fluid absorbing materials used in the present invention will have the capacity of absorbing in the range from its own weight up to, but not limited to, 50 times its own weight.

The fluid-absorbing materials of this invention may also be capable of holding not only the fluids themselves, but also the solutes dissolved within the fluids. Thus, for example, excess fluids that accumulate in the body may not only be water, but also a mixture of water and other chemicals or solutes, such as potassium ions. Accordingly, the fluid absorbing materials released by the capsules of this invention should be capable of entrapping not only water but also the solutes that are dissolved therein.

Without being bound to any particular mechanistic theory of action, the mixture of water and chemicals and/or solutes enter porous areas within the polymer and are consequently entrapped within. The entrapment of the water, chemical, and/or solutes may operate by a non-chemical or physical action known as three-dimensional entrapment. The fluid absorbing material allows for three dimensional entrapment due to its high swelling capacity (also known as water regain). Accordingly, any solutes that are dissolved within the fluids will therefore be entrapped, helping in the removal of various serum toxins, ions, and chemicals from subject suffering from various diseases associated with fluid retention.

The fluid-absorbing materials of the present invention may include those materials which are added to remove or retain toxic ion such as ion exchange materials. For example, it is well known that polystyrene sulfonate sodium (PSS) sold as KAYEXALATE® has been used in the treatment of hyperkalemia. However, the use of PSS in the treatment of hyperkalemia is often challenging due to the technical complications associated with absorption of other cations. Accordingly, the capsules of the present invention may used to overcome this technical hurdle. For example, the capsules may be designed to encapsulate the fluid-absorbing material mixed with PSS in a timed or targeted delivery system to overcome the premature absorption of other cations. Other toxic ion removing materials may include the combination of PSS and sorbitol for the treatment of hyperkalemia; and lactulose for the treatment of hyperammonemia and uremia. The polymers used in the present invention are readily available from a variety of commercial sources and suppliers.

As can be appreciated from the foregoing, the insoluble hydrophilic, cross-linked polymers, such as polysaccharides, according to the invention must be able to take up water in the gastrointestinal system without being absorbed and circulated into the body proper through the walls of the intestinal lumen. Further, the insoluble hydrophilic, cross-linked polymers, such as polysaccharides, of the present invention must be non-toxic, reasonably palatable, and non-digestible.

As a preferred embodiment of the invention, the insoluble hydrophilic, cross-linked polysaccharides which are used in the present invention are hydroxyl-group containing substances, both ionic and non-ionic, preferably modified dextrans. The use of these insoluble hydrophilic cross-linked polysaccharides are fully disclosed in US Patent 4,470,975. In addition to dextrans, other hydroxyl-group containing insoluble hydrophilic cross-linked polysaccharides which are useful in the present invention include modified starches, dextrin, cellulose, and polyglucose, and hydroxyl-group containing charged or uncharged derivatives of these substances or products obtained by a partial depolymerization of the same, as well as fractions thereof. In one embodiment, dextrans may be modified such that they are obtained by cross-linking hydrophilic dextrans with epichlorohydrin into insoluble forms that retain the hydrophilic nature of dextrans but is depleted of ionic groups.

The dextran or other polysaccharide macromolecules are typically modified by cross-linking to give a three-dimensional network of polysaccharide chains. Because of their high content of hydroxyl groups, these cross-linked polysaccharides are strongly hydrophilic and swell considerably in water. Various types of insoluble, hydrophilic, cross-linked polysaccharides are available, differing in their swelling properties. The degree of swelling is an identifying characteristic of these hydrophilic polysaccharides. The degree of swelling reflects differences in the degree of cross-linkage of the polysaccharides. As is well known in the art, the transport of most organic molecules through the walls of the intestinal lumen requires an active process, and it does not appear that such a process exists for the insoluble hydrophilic, cross-linked polysaccharides of the present invention.

The preparation of the insoluble, hydrophilic, cross-linked polysaccharides used in the present invention is fully described in a series of British and United States patents assigned to Aktiebolaget Pharmacia, a Swedish company. These patents are:

| **British Patent** | **U.S. Patent No.** |
|---|---|
| - | 3,002,823 |
| 854,715 | 3,042,667 |
| 936,039 | 3,275,576 and 3,277,025 |
| 974,054 | 3,208,994 |
| 1,013,585 | - |

It is to be understood that the disclosures of the above-listed British and United States patents are related, but are not necessarily identical. For example, although Examples 1 to 14 of U.S. Pat. No. 3,042,667 are virtually identical to Examples 4 to 18 of British Pat. No. 854,715, the disclosure and claims of the British patent include both water soluble and water insoluble hydrophilic cross-linked dextrans, whereas the United States patent disclosure is limited to water-insoluble hydrophilic cross-linked dextrans. The hydrophilic, cross-linked polysaccharides used in the present invention are all of the water-insoluble type. The disclosures of the five United States patents listed above enable one of ordinary skill in the art to prepare the insoluble, hydrophilic, cross-linked polysaccharides useful in the present invention. The four British patents listed above, as well as the disclosures of U.S. Pat Nos. 3,300,474 and 3,542,759 are referred to for additional disclosure of insoluble hydrophilic, cross-linked polysaccharides useful in the present invention.

The preferred insoluble hydrophilic, cross-linked polysaccharides used in the present invention are copolymerization products in the form of gel grains comprising a three-dimensional macroscopic network of dextran substances, built up of chains of mainly alpha-1,6-glucosidically bonded glucose residues, bonded together by ether bridges of the general type --R--O--X--O--R--, wherein R represents the dextran substances and X is an aliphatic radical containing from 3 to 10 carbon atoms, the copolymerization product being water-insoluble but being capable of absorbing water with swelling, the water regain of the product being within the range of about 1 to 50 grams per gram of the dry gel product. The "water regain" of the dry gel product is the amount of water in grams which can be absorbed by one gram of the dry gel. The capacity of swelling of the gelled product may be measured in terms of the water regain. While water regain in the range of about 1 to about 50 grams of water per gram of dry gel product is preferred, dry gel products exhibiting a water regain in the range of about 1 to about 55 grams; 1 to about 60, 1 to about 65 grams, 1 to about 70 grams, 1 to about 75 grams, 1 to about 80 grams, grams 1 to about 85 grams, 1 to about 90 grams, 1 to about 95 grams, or 1 to about 100 grams of water, or even more, are useful in the practice of the present invention. Indeed, it will be understood that the maximum water regain of the product is limited by the ability of the cross-linked polysaccharides used in the present invention to resist degradation in the gastrointestinal system.

In general, the typical process for preparing the preferred insoluble hydrophilic, cross-linked dextrans used in the present invention can be characterized as a block polymerization process in which a substituted dextran is reacted with a bifunctional organic substance containing halogen and/or epoxy groups, capable of reacting with the hydroxyl groups of the substituted dextran to form ether-linkages. The reaction is conducted in the presence of an alkaline substance which may function either as an acceptor for the hydrohalide liberated as a result of the reaction (when the reaction that forms the basis of the ether-formation is a condensation in which a hydrohalide is split off), or the alkaline substance may act as a catalyst when the reaction is a pure reaction of addition. The block copolymers thus formed are insoluble in water, but capable of swelling therein. Examples of suitable alkaline substances are the alkali metal hydroxides, preferably sodium hydroxide and the alkaline earth metal hydroxides, and also tertiary amines and quaternary ammonium hydroxides.

The block polymerization process takes place in the presence of water and is catalyzed by the alkaline substances described above. As previously stated, all essential details of the preparation of the insoluble hydrophilic, cross-linked dextrans and other polysaccharides used in the present invention are set forth in the five United States patents incorporated by reference herein. Additional details of the preparation of the hydrophilic cross-linked polysaccharides useful in the present invention may be found in four British patents and in U.S. Pat. Nos. 3,300,474 and 3 ,542,759.

The preferred insoluble, hydrophilic, cross-linked dextrans of the present invention were originally developed and sold by Pharmacia Fine Chemicals, Inc., and are now products of G.E. Healthcare Biosciences, and are sold by G.E. Healthcare Biosciences, 800 Centennial Avenue, Piscataway, N.J. 08854 under the trademark Sephadex. Sephadex-brand insoluble, hydrophilic, cross-linked dextrans are available from G.E. Healthcare Biosciences subsidiaries or representatives in most countries of the world. A list of suppliers may be obtained by writing directly to G.E. Healthcare Biosciences, Piscataway, N.J.

### EXAMPLES

### Example 1

Five normal Sprague-Dawley rats were placed in metabolic cages and urine output on standard rat chow and free access to water measured for a period of five days. During this period, the mean urinary excretion rate in milliliters per day for the entire group was 14.72±0.95 (standard error of mean, hereinafter SEM). The animals were subsequently given Sephadex (G-50) mixed with food in equal proportions. They were maintained on this regimen for an additional 7 days. During this latter treatment the mean daily urinary excretion rate was 3.5±0.48 (SEM) ml per day, a highly significant difference.

### Example 2

Six Sprague-Dawley rats were divided into 2 groups of 3 each. One group was given regular or standard rat chow and water ad lib and the second group was given the rat chow ground and mixed with equal parts of Sephadex (G 50) and permitted water ad lib. These studies were carried out for a period of 9 days. During this 9 day period the average daily water intake for the group that received no Sphadex was 30.5 ml per day (±1.6 ml SEM). During the same time, they excreted an average daily urine output of 8.54±0.66 ml per day. In contrast, the group receiving Sephadex ingested more water, an average daily intake of 46.72±2.0 ml per day but put out no measurable quantity of urine. For a subsequent 4 day period, the Sephadex was discontinued and on day 3 and 4 of this last period water intake and urine output for the 2 groups was indistinguishable.

### Example 3

The quantity of water excreted by way of the feces was measured in 10 animals. Ten male Sprague-Dawley rats were given Sephadex G 100 mixed in 1 to 1 ratio with pulverized purina rat chow. In 6 of the animals the weight of the feces excreted per day was determined. In 4 animals the water content of the feces was measured. The results of the study are shown in Figures 1 and 2. Fecal excretion per animal on the control day was 7 grams and the water content 65%. Thus, at the beginning of the study the animals were excreting approximately 4.5 ml of water in the feces. This rose steadily during this study so that by day 7 the animals were excreting 20 grams of feces per day per animal and the water content had risen to 90%. Thus, the fecal water loss after 7 days on the Sephadex regimen was 18 ml per day, an increased fecal water loss of more than four-fold. This increased water loss in the feces was even greater than average daily urinary excretion volumes in control animals cited in experiment 2 above. Thus the combination of examples of 1, 2 and 3 demonstrate that the sharp diminution and disappearance of urine production is associated with a comparable increase in water loss from the gastrointestinal tract.

### Example 4

Rats with non-functioning kidneys.

Sixteen male Sprague-Dawley rats had their kidney function terminated abruptly by urethral ligation. The rats were then divided into a control group of 4 animals and an experimental group of 12 animals. To ensure that all animals took in a constant quantity of either Sephadex (G50) or a placebo, the Sephadex was suspended in mineral oil and given to the experimental or treatment group. A comparable mixture of mineral oil placebo was given to the control group of animals. The results of the study are shown in Figure 3. A striking difference in survival time was noted. All control animals were dead in less than 40 hours whereas the experimentally treated group survived for more than 100 hours. (Mean survival time: experimental group 103.25±10 hours; survival time for control group 36.25±1.25 hours). During the study period, the water intake in these animals did not differ significantly. The control group took in an average of 0.73±0.11 ml of water per hour and the group treated with Sephadex (G50) took an average of 0.58±0.04 ml of water per hour. Six of the treated animals survived 5 days and one animal survived for 7 days. The treated group received 1.4 grams of Sephadex per day per animal for the duration of their survival. The control group received only placeboes of mineral oil. All animals were permitted food and water ad lib for the duration of their survival. Another group of 8 animals operated upon at a different time were submitted to uretheral ligation to serve as controls. All of these animals died between the first and second day.

Thus the addition of Sephadex to the regimen of these rats without any kidney function resulted in an increase in mean survival time of more than three-fold demonstrating that Sephadex exerts an important beneficial effect upon the length of survival in uremia. The information contained in the 3 preceding examples and the present one demonstrates that Sephadex when given orally leads to profound reduction in the rate of urine formation and produces a comparable increase in the quantity of water excreted in the feces. Further, when given to rats rendered uremic by uretheral ligation (thereby preventing any urine excretion) it increases the survival time three-fold. On the basis of this information, it would be expected to behave in the same manner when used in humans without renal function.

### Example 5

Six Sprague-Dawley rats were maintained under control conditions in metabolic cages for a 5 day period during which time food intake was weighed carefully while they were permitted food ad lib. They were also permitted free access to water. During this control period, the daily food intake averaged 20.69±0.82 (SEM) grams per day. Beginning on day 6, five of the animals were given food mixed with 50% Sephadex (G 50) and a sixth animal served as a control to document weight changes in an animal not receiving Sephadex during this period. Balance studies were continued throughout this period and daily food intake measured. The results of this study are shown in Figure 4. The grams of food ingested by the group of animals receiving Sephadex fell to 11.4±0.95 grams per day, a 50% reduction in food intake. During this period of time, the average body weight of the group receiving Sephadex dropped from 330 grams to 290 grams, an average weight loss per animal of 40 grams or more than 10% of the body weight. The lone control animal gained 25 grams during the same period and during that period he continued to ingest an average daily weight of food of 27.1±1.98 grams.

In a second but somewhat shorter experiment a group of 27 animals were divided into a group of 10 control animals and 17 animals receiving Sephadex (G 50). The animals were kept in metabolic cages; the control animals were given Purina rat chow and water ad lib and the Sephadex animals received their food as a 50% mixture of Sephadex (G 50) in pulverized Purina rat chow. During this study the 17 animals receiving Sephadex lost an average of 5 grams of body weight whereas the control group gained an average of 12 grams. The results of this study are shown in Figure 5. These studies provide evidence that the daily ingestion of Sephadex is associated with the corresponding reduction in caloric or food intake.

### Example 6

Figure 6A illustrates an experiment demonstrating the effect of treatment with three different insoluble, hydrophilic, cross-linked dextrans according to the present invention. A group of two rats were given a diet which consisted of 50% by weight Sephadex G-50 and 50% normal rat chow. Another group of 2 rats were given a diet which consisted of 50% by weight Sephadex G-100 and 50% normal rat chow. A third group consisting of a single rat was given a diet which consisted of 50% by weight of Sephadex G-200 and 50% normal rat chow. A control group consisting of a single rat was given a normal diet consisting entirely of normal rat chow.

From Figure 6A, it can be seen that in each case treatment with an insoluble, hydrophilic, cross-linked dextran according to the present invention resulted in a substantially reduced volume of urine, compared to the volume of the control group. At the same time, it can be seen from Figure 6B that the water content of the feces of rats given a diet containing an insoluble hydrophilic, cross-linked dextran according to the present invention is significantly higher than the water content of the feces of the control group. Both groups were permitted to consume as much water as they desired, and their actual water consumption is shown in the table below:

| **DAILY VOLUME OF WATER CONSUMED (IN MILLILITERS)** | | | | |
|---|---|---|---|---|
| **Day** | **Control** | **50% G-50** | **50% G-100** | **50% G-200** |
| 1 | 52 | 32 | 18 | 41 |
| 2 | 46 | 30 | 42 | 33 |
| 3 | 42 | 24 | 40 | 0 |
| 4 | 30 | 35 | 55 | 52 |
| 5 | 40 | 58 | 48 | 50 |
| 6 | 50 | 65 | 69 | 60 |
| 7 | 50 | 60 | 75 | 50 |

### Example 7

Figures 7A and 7B illustrate the results of an experiment demonstrating the effects of varying the amount of preferred insoluble, hydrophilic, cross-linked dextrans in the diets of rats. A group of 2 rats were given a diet which consisted of 10% by weight Sephadex G-100 and 90% by weight normal rat chow. Another group of 2 rats were given a diet which consisted of 25% by weight Sephadex G-100 and 75% by weight normal rat chow. A third group of 2 rats were given a diet consisting of 50% by weight Sephadex G-100 and 50% by weight normal rat chow. Each group was given this diet for a period of 5 consecutive days, and then each group was transferred to a normal diet consisting entirely of rat chow. After a period of four days of a normal diet, each group was again tested. Figure 7A illustrates the results of the varying diets on the daily volume of urine of each group. It will be noted that in every case, the volume of urine of rats consuming a diet including an insoluble, hydrophilic, cross-linked dextran according to the present invention is substantially less than the volume of urine of the same group on a normal diet. It will further be observed that in every case, as the concentration of an insoluble, hydrophilic, cross-linked dextran is increased, the volume of urine in the treated group decreases. That is, the average daily volume of urine of rats given a diet including 25% by weight Sephadex G-100 is significantly less than the daily volume of urine of rats given a diet containing only 10% by weight Sephadex G-100. Similarly, the daily volume of urine of rats given a diet consisting of 50% by weight Sephadex G-100 is significantly less than the daily volume of urine of rats given a diet containing only 25% by weight Sephadex G-100. Thus, it may be seen that the effect of an insoluble, hydrophilic, cross-linked dextran according to the present invention in substantially reducing the daily volume of urine, is dependent on the dose of dexttran from 10% of the diet at least to 50% of the diet. FIG. 7B illustrates the water content of the feces of each of these groups of rats, again compared to the water content of the feces of the same rats fed a normal diet. In every case, it will be noted that the effect of treatment with an insoluble, hydrophilic, cross-linked dextran according to the present invention was to increase the water content of the feces of rats so treated, compared to rats consuming a normal diet. In every case, each rat was permitted to consume as much water as desired. The actual water consumed by each group of rats is listed in the table below:

| **DAILY VOLUME OF WATER CONSUMED (IN MILLILITERS)** | | | |
|---|---|---|---|
| **Day** | **10% G-100** | **25% G-100** | **50% G-100** |
| Sephadex-Containing Diet | | | |
| 1 | 68 | 103 | 73 |
| 2 | 56 | 81 | 73 |
| 3 | 69 | 69 | 73 |
| 4 | 61 | 74 | 78 |
| 5 | 55 | 78 | 67 |

| Normal Diet | | | |
|---|---|---|---|
| 1 | 45 | 52 | 45 |
| 2 | 53 | 46 | 55 |
| 3 | 45 | 42 | 54 |

These experiments show that diets containing insoluble, hydrophilic, cross-linked carbohydrates according to the present invention are able to divert the mode of water elimination from the renal route to the gastrointestinal route, and remove water from the body by the gastrointestinal route. These pharmacological properties are of significant therapeutic value in the treatment of edema, water intoxication in chronic renal failure, and in the treatment of other forms of fluid retention such as congestive heart failure, cirrhosis of the liver and other disorders associated with refractory swelling. These pharmacological properties are also useful as a means of reducing caloric intake, in the treatment of conditions such as obesity. Although the above experiments were conducted with animals other than human beings, preliminary experiments indicate that the insoluble, hydrophilic, cross-linked carbohydrates according to the present invention exhibit the same pharmacological properties when used to treat human beings as in the treatment of other animals.

### Example 8

To produce the fluid absorbing material of the present invention, 500 g. of dextran (Mw-1,800,000) is dissolved in 2 liters of an aqueous solution of sodium hydroxide and 100 g. of epichlorohydrin. After 1 hour at 45 degrees Celsius, a gel is formed which is cured by heating to 45 degrees for 24 hours. After grinding, neutralization, washing and substantial drying, 540 g. of the product with a water regain of 10g./g. of the dry product is obtained. The product is (i) ground to a particle size ranging between 50 and 200 mesh, (ii) neutralized with hydrochloric acid, (iii) washed with water on a filter until free of salt and (iv) dried to a constant weight in an oven at 80 degrees Celsius. Each resulting bead contains hundreds of microscopic pores and channels through which water, chemicals, and/or solutes may become entrapped.

### Example 9

Vcap® and Vcap® plus capsules are filled with Sephadex G-50 or the crossed linked dextrans described in Example 8. The capsule is capable of pH independent dissolution and therefore allows even distribution of the Sephadex G-50 or the crossed linked dextrans described in Example 8 along the entire gastrointestinal tract. The water insoluble Sephadex G-50 or the crossed linked dextrans described in Example 8 contained in the capsule is released and begins to absorb fluid present within the stomach and/or intestine..

The Vcap® and Vcap® plus capsules contain approximately 2000 mg of Sephadex G-50 or the crossed linked dextrans described in Example 8. Accordingly, a patient receiving the capsules containing the Sephadex-G50 or the crossed linked dextrans described in Example 8 consumes approximately 10 capsules per day for a total of approximately 20,000 milligrams of the Sephadex G-50 or the crossed linked dextrans described in Example 8. This amount will absorb approximately 100 milliliters of excess fluid.

The effectiveness may be measured by determining the urine output before and after consumption of the fluid absorbing material containing capsules. Alternatively, the effectiveness of the capsule may be measured by determining the overall water content in the feces both before and after consumption of the capsules. The effectiveness of the oral dosing may be adjusted up or down based on the overall effectiveness of removing excess liquid from the patient.

Alternatively, Vcap® and Vcap® plus capsules are filled with Sephadex G-100. Accordingly a patient receiving the capsules containing Sephadex G-100 consumes approximately 10 capsules per day for a total of approximately 20,000 milligrams of the Sephadex G-100. This amount will absorb approximately 200 milliliters of excess fluid. The overall effectiveness of the consumed capsules will be measured and determined as outlined above.

### Example 10

NPcaps® are filled with either Sephadex G-50 or Sephadex G-100. NPcaps provide the advantage of quick disintegration. Accordingly, patients requiring the immediate release of either Sephadex G-50 or Sephadex G-100 into the stomach will benefit from this type of capsule. Patients consume approximately 10 capsules per day for a total of approximately 20,000 milligrams per kilogram of body weight. The overall effectiveness of the treatment using NPcaps® containing Sephadex G-50 or Sephadex G-100 can determined as discussed in Example 8.

### Example 11

Patients suffering from acute renal failure are administered either the Sephadex G-50, the crossed linked dextrans described in Example 8, or Sephadex G-100 capsules described in Examples 9 and 10. All patients receiving the capsules are dosed based on 20,000 milligrams Sephadex containing capsules. Alternatively, those patients suffering from more severe renal failure are administered larger dosages of Sephadex containing capsules. Those of skill in the art will be able to chose the appropriate dosing based on the amount of excess fluid removal required.

Patients put on a regimen of daily oral ingestion of capsules containing the Sephadex will exhibit a significant reduction in the excretion of urine output and an increase in the expulsion of water through the fecal matter. Additionally, patients receiving the oral capsules comprising Sephadex will exhibit improvements in the symptoms associated with renal failure.

## Claims

1. An orally consumable medical product comprising a capsule constructed of substantially nitrogen-free material, wherein substantially nitrogen-free means comprising less than 4% nitrogen, wherein the capsule contains a water-insoluble fluid-absorbing material having the capacity of absorbing in the range from its own weight up to 50 times its own weight or more.

2. A product of claim 1, wherein the capsules are made from non-animal and/or non-protein based material, wherein the materials contain no protein.

3. The product of claim 1, wherein the capsule comprises a singular opening, preferably at the distal end of the capsule, sealed with a hydrogel plug, optionally made of polymethacrylates, hydropropylmethyl cellulose, polyvinyl alcohol, polyvinyl acetate, polyethylene oxide, saturated polyglycolated glycerides, glyceryl monooleates, pectin, sodium polyacrylate, acrylate polymers, copolymers with an abundance of hydrophilic groups, agarose, methylcellulose, hyaluronan, and other naturally derived polymers, and preferably wherein the hydrogel plug swells, erodes, congeals, melts, enzymatically erodes, or a combination thereof.

4. The product of one of the claims 1-3, wherein release of the water insoluble fluid-absorbing material from the capsule is pH dependent, preferably at a pH of 1 to 3, or preferably at a pH of 5 to 8.

5. The product of one of the claims 1 or 4, wherein the capsule is a two piece capsule.

6. The product of claim 1, wherein the capsule is constructed of
- a natural vegetable-derived polysaccharide, preferably being hypromellose, cellulose, alginate, or a biodegradable carbohydrate, or
- rubber, silicone rubber, gelatinous material, or a combination thereof.

7. The product of claim 1, wherein the fluid-absorbing material is a hydrophilic fiber, powder, gel, or grain; or wherein the fluid-absorbing material is an insoluble, cross-linked polysaccharide, preferably nitrogen-free in pH ranging from about 1 to about 8; or wherein the fluid-absorbing material is non-ionic and further comprises a high content of hydroxyl groups.

8. The product of one of claims 1-7, wherein the fluid-absorbing material is dextran, preferably a modified dextran, wherein the modified dextran is preferably cross linked with epichlorohydrin; modified starches; dextrin; cellulose; polyglucose; or product obtained by partial depolymerization; or combination thereof.

9. The product of claim 1, wherein the fluid-absorbing material is a copolymer in the form of a gel grain comprising a three dimensional network of dextran substances, preferably a copolymer comprising α,-1,6-glucosidically bonded glucose residues, preferably having general formula -R-O-Xₙ₋O-R, wherein R is a dextran substance, X is an aliphatic radical with carbon, n is 3-10; and preferably wherein the α-1,6-glucosidically bonded glucose residues are linked by ether bridges.

10. The product of claim 1, wherein the fluid-absorbing material comprises a water regain in the range of 1 to 100 grams/dry gram of dry product, preferably 1 to 85 grams/dry grams of dry product and optionally wherein the fluid-absorbing material is a particle size ranging from 50 to 200 mesh.

11. A method of preparing an orally consumable medical product according to any one of claims 1 to 11, said method comprising encapsulating a fluid absorbing polymer comprising:
a. filling a capsule constructed of substantially nitrogen-free material, wherein substantially nitrogen-free means comprising less than 4% nitrogen, with a fluid absorbing material; and
b. sealing or enclosing the capsule, whereby the capsule encapsulates the fluid absorbing material.

12. A capsule constructed of substantially nitrogen-free material, wherein substantially nitrogen-free means comprising less than 4% nitrogen, wherein the capsule contains a water-insoluble, fluid-absorbing material for use as a medicament for treating a disease or disorder selected from renal failure, obesity, constipation, hyperkalemia, uremia, hyperammonemia, non-insulin dependent diabetes or combinations thereof.

13. A capsule constructed of substantially nitrogen-free material, wherein substantially nitrogen-free means comprising less than 4% nitrogen, wherein the capsule contains a water-insoluble, fluid-absorbing material for use according to claim 12 for inducing a sense of fullness, reducing the frequency of dialysis, removing excess accumulation of serum toxins, or reducing triglycerides.

## Patentansprüche

1. Oral einnehmbares medizinisches Produkt, das eine Kapsel umfasst, die aus im Wesentlichen stickstofffreiem Material gebildet ist, wobei im Wesentlichen stickstofffrei bedeutet, dass sie weniger als 4 % Stickstoff umfasst, wobei die Kapsel ein in Wasser unlösliches fluidabsorbierendes Material aufweist, das in der Lage ist, im Bereich von seinem Eigengewicht bis zum 50-Fachen seines Eigengewichts zu absorbieren.

2. Produkt nach Anspruch 1, wobei die Kapseln aus nicht-tierischem und/oder nicht auf Protein basierendem Material gefertigt sind, wobei die Materialien kein Protein enthalten.

3. Produkt nach Anspruch 1, wobei die Kapsel eine einzige Öffnung, vorzugsweise am distalen Ende der Kapsel umfasst, die mit einem Hydrogelstopfen abgedichtet ist, der optional aus Polymethacrylaten, Hydropropylmethylcellulose, Polyvinylalkohol, Polyvinylacetat, Polyethylenoxid, gesättigtem polyglycolierten Glyceriden, Glycerylmonooleaten, Pektin, Natriumpolyacrylat, Acrylatpolymeren, Copolymeren mit einem Überschuss an hydrophilen Gruppen, Agarose, Methylcellulose, Hyaluronan und anderen natürlich gewonnenen Polymeren gefertigt ist, und wobei der Hydrogelstopfen aufquillt, erodiert, geliert, schmilzt, enzymatisch erodiert oder eine Kombination davon.

4. Produkt nach einem der Ansprüche 1-3, wobei eine Freisetzung des in Wasser unlöslichen fluidabsorbierenden Materials aus der Kapsel pH-abhängig ist und vorzugsweise bei einem pH von 1 bis 3 oder vorzugsweise bei einem pH von 5 bis 8 stattfindet.

5. Produkt nach einem der Ansprüche 1 oder 4, wobei die Kapsel eine zweiteilige Kapsel ist.

6. Produkt nach Anspruch 1, wobei die Kapsel gebildet ist aus
- einem natürlichen, aus Pflanzen gewonnenen Polysaccharid, bei dem es sich vorzugsweise um Hypromellose, Cellulose, Alginat oder ein biologisch abbaubares Kohlehydrat handelt oder
- Kautschuk, Silikonkautschuk, gelatineartigem Material oder einer Kombination davon.

7. Produkt nach Anspruch 1, wobei das fluidabsorbierende Material eine hydrophile Faser, ein Pulver, ein Gel oder ein Korn ist; oder wobei das fluidabsorbierende Material ein unlösliches, vernetztes Polysaccharid ist, vorzugsweise stickstofffrei in einem pH im Bereich von etwa 1 bis etwa 8; oder wobei das fluidabsorbierende Material nichtionisch ist und ferner einen hohen Anteil an Hydroxylgruppen aufweist.

8. Produkt nach einem der Ansprüche 1-7, wobei das fluidabsorbierende Material ist:
Dextran, vorzugsweise modifiziertes Dextran, wobei das modifizierte Dextran vorzugsweise mit Epichlorhydrin vernetzt ist; modifizierte Stärken; Dextrin; Cellulose; Polyglucose; oder
ein Produkt, das durch eine Teil-Depolymerisation erhalten wird; oder eine Kombination davon.

9. Produkt nach Anspruch 1, wobei das fluidabsorbierende Material ein Copolymer in Form eines Gelkorns ist, das ein dreidimensionales Netzwerk von Dextransubstanzen umfasst, vorzugsweise ein Copolymer, das α-1,6-glucosidisch gebundene Glucosereste umfasst, vorzugsweise mit der allgemeinen Formel -R-O-Xₙ-O-R, wobei R eine Dextransubstanz ist, X ein aliphatischer Rest mit Kohlenstoff ist, n 3-10 ist; und wobei die α-1,6-glucosidisch gebundenen Glucosereste vorzugsweise durch Etherbrücken verknüpft sind.

10. Produkt nach Anspruch 1, wobei das fluidabsorbierende Material eine Wasserrückgewinnung im Bereich von 1 bis 100 Gramm/Trockengramm trockenes Produkt, vorzugsweise 1 bis 85 Gramm/Trockengramm trockenes Produkt aufweist, und wobei das fluidabsorbierende Material optional eine Teilchengröße im Bereich von 50 bis 200 Mesh aufweist.

11. Verfahren zum Herstellen eines oral einnehmbaren medizinischen Produkts nach einem der Ansprüche 1 bis 11, wobei das Verfahren das Verkapseln eines fluidabsorbierenden Polymers umfasst, welches umfasst:
a. Füllen einer Kapsel, die aus im Wesentlichen stickstofffreiem Material gebildet ist, wobei im Wesentlichen stickstofffrei bedeutet, dass es weniger als 4 % Stickstoff enthält, mit einem fluidabsorbierenden Material; und
b. Abdichten oder Verschließen der Kapsel, wobei die Kapsel das fluidabsorbierende Material einschließt.

12. Kapsel, die aus im Wesentlichen stickstofffreiem Material gebildet ist, wobei im Wesentlichen stickstofffrei bedeutet, dass sie weniger als 4 % Stickstoff enthält, wobei die Kapsel ein in Wasser unlösliches, fluidabsorbierendes Material zur Verwendung als Medikament zur Behandlung einer Krankheit oder Gesundheitsstörung umfasst, die ausgewählt ist aus Nierenversagen, Fettleibigkeit, Verstopfung, Hyperkaliämie, Urämie, Hyperammonämie, nicht-insulinabhängigem Diabetes oder Kombinationen davon.

13. Kapsel, die aus im Wesentlichen stickstofffreiem Material gebildet ist, wobei im Wesentlichen stickstofffrei bedeutet, dass sie weniger als 4 % Stickstoff umfasst, wobei die Kapsel ein in Wasser unlösliches, fluidabsorbierendes Material zur Verwendung nach Anspruch 12 umfasst, um ein Sättigungsgefühl hervorzurufen, eine Dialysefrequenz zu senken, eine übermäßige Ansammlung von Serumtoxinen zu beseitigen oder Triglyceride zu verringern.

## Revendications

1. Produit médical consommable par voie orale comprenant une capsule construite à partir d'une matière sensiblement dépourvue d'azote, dans lequel sensiblement dépourvue d'azote signifie comprenant moins de 4 % d'azote, dans lequel la capsule contient une matière absorbant un fluide insoluble dans l'eau ayant la capacité d'absorption dans la plage de son propre poids jusqu'à 50 fois son propre poids ou plus.

2. Produit selon la revendication 1, dans lequel les capsules sont constituées d'une matière non animale et/ou non protéique, dans lequel les matières ne contiennent pas de protéine.

3. Produit selon la revendication 1, dans lequel la capsule comprend une ouverture singulière, de préférence à l'extrémité distale de la capsule, obturée avec un bouchon d'hydrogel, facultativement constituée de polyméthacrylates, d'hydropropylméthyl cellulose, de poly(alcool vinylique), de poly(acétate de vinyle), de poly(oxyde d'éthylène), de poly(glycérides glycolés), de monooléates de glycéryle, de pectine, de poly(acrylate de sodium), de polymères d'acrylate, de copolymères avec une abondance de groupes hydrophiles, d'agarose, de méthylcellulose, de l'hyaluronane, et d'autres polymères naturels, et de préférence dans lequel le bouchon d'hydrogel gonfle, s'érode, se congèle, fond, s'érode enzymatiquement, ou l'une de leur combinaison.

4. Produit selon l'une des revendications 1 à 3, dans lequel une libération de la matière absorbant un fluide insoluble de la capsule est dépendante du pH, de préférence à un pH de 1 à 3, ou de préférence à un pH de 5 à 8.

5. Produit selon l'une des revendications 1 ou 4, dans lequel la capsule est une capsule à deux pièces.

6. Produit selon la revendication 1, dans lequel la capsule est construite à partir de
- un polysaccharide dérivé d'un végétal naturel, qui est de préférence l'hypromellose, la cellulose, l'alginate, ou un glucide biodégradable, ou
- le caoutchouc, un caoutchouc de silicone, une matière gélatineuse, ou l'une de leur combinaison.

7. Produit selon la revendication 1, dans lequel la matière absorbant un fluide est une fibre hydrophile, une poudre, un gel, ou un grain ; ou dans lequel la matière absorbant un fluide est un polysaccharide réticulé insoluble, de préférence dépourvu d'azote dans une plage de pH allant d'environ 1 à environ 8 ; ou dans lequel la matière absorbant un fluide est non ionique et comprend en outre une haute teneur en groupes hydroxyle.

8. Produit selon l'une des revendications 1 à 7, dans lequel la matière absorbant un fluide est le dextrane, de préférence un dextrane modifié, dans lequel le dextrane modifié est de préférence réticulé avec de l'épichlorohydrine ; des amidons modifiés ; de la dextrine ; de la cellulose ; du polyglucose ; ou un produit obtenu par dépolymérisation partielle ; ou l'une de leur combinaison.

9. Produit selon la revendication 1, dans lequel la matière absorbant un fluide est un copolymère sous la forme d'un grain de gel comprenant un réseau tridimensionnel de substances de dextrane, de préférence un copolymère comprenant des résidus de glucose liés α-1,6-glucosidiquement, de préférence répondant à la formule générale -R-O-Xₙ-O-R, dans laquelle R est une substance de dextrane, X est un radical aliphatique avec carbone, n vaut 3 à 10 ; et de préférence dans lequel les résidus de glucose liés α-1,6-glucosidiquement sont liés par des ponts éther.

10. Produit selon la revendication 1, dans lequel la matière absorbant un fluide comprend un regain d'eau dans la plage de 1 à 100 grammes/gramme sec de produit sec, de préférence 1 à 85 grammes/gramme sec de produit sec et facultativement dans lequel la matière absorbant un fluide a une taille de particule allant de 50 à 200 mesh.

11. Procédé de préparation d'un produit médical consommable par voie orale selon l'une quelconque des revendications 1 à 11, ledit procédé comprenant l'encapsulation d'un polymère absorbant un fluide comprenant :
a. le remplissage d'une capsule construite à partir d'une matière sensiblement dépourvue d'azote, dans lequel sensiblement dépourvue d'azote signifie comprenant moins de 4 % d'azote, avec une matière absorbant un fluide ; et
b. l'obturation ou la fermeture de la capsule, moyennant quoi la capsule encapsule la matière absorbant un fluide.

12. Capsule construite à partir d'une matière sensiblement dépourvue d'azote, dans lequel sensiblement dépourvue d'azote signifie comprenant moins de 4 % d'azote, dans laquelle la capsule contient une matière absorbant un fluide, insoluble dans l'eau pour utilisation comme médicament destiné à traiter une maladie ou un trouble choisi(e) parmi une insuffisance rénale, l'obésité, la constipation, l'hyperkaliémie, l'urémie, l'hyperammoniémie, le diabète non-insulino dépendant ou leurs combinaisons.

13. Capsule construite à partir d'une matière sensiblement dépourvue d'azote, dans lequel sensiblement dépourvue d'azote signifie comprenant moins de 4 % d'azote, dans laquelle la capsule contient une matière absorbant un fluide, insoluble dans l'eau, pour utilisation selon la revendication 12 en vue d'induire une sensation de plénitude, de réduire la fréquence de dialyse, d'éliminer l'accumulation en excès de toxines sériques, ou de réduire les triglycérides.
